# EUROPEAN PATENT APPLICATION

(11) **EP 3 667 609 A1**
(43) Date of publication of application: **17.06.2020**
(21) Application number: 19204868.4
(22) Date of filing: 23.10.2019
(51) Int. Cl.: G06T 5/50, G06T 7/11, G06K 9/62

(54) **METHOD AND APPARATUS FOR RECONSTRUCTING MEDICAL IMAGE**

(30) Priority: 11.12.2018 KR 20180159117
(71) Applicant: Medicalip Co., Ltd., Gangwon-do 24341 (KR)
(72) Inventor: Park, Sang Joon, 04595 Seoul (KR); Lee, Doo Hee, 03075 Seoul (KR); Yoon, Soon Ho, 06092 Seoul (KR)
(74) Representative: Patentanwälte Ruff, Wilhelm, Beier, Dauster & Partner mbB

(57) **Abstract**

Provided is a method and apparatus for reconstructing a medical image. The apparatus for reconstructing a medical image generates at least one base image by reducing a dimensionality of a three-dimensional (3D) medical image, generates at least one segmented image by reducing a dimensionality of a 3D image of a region of a tissue segmented from the 3D medical image or a 3D image of a region excluding the tissue from the 3D medical image, and trains, by using training data including the at least one base image and the at least one segmented image, an artificial intelligence (AI) model that separates at least one tissue from a medical image showing a plurality of tissues overlapping one another on the same plane.

## Description

The invention relates to a method and to an apparatus for reconstructing a medical image.
This application claims the benefit of Korean Patent Application No. 10-2018-0159117, filed on December 11, 2018, in the Korean Intellectual Property Office, the disclosure of which is incorporated herein in its entirety by reference.

X-ray radiograph has been applied to a medical field since discovery of X-rays by Roentgen in 1895 and is the most widely used radiographic testing method that allows a clinician to evaluate a patient by creating images of different parts of a body. When X-ray radiographs are captured of a part of a patient's body to be examined, X-rays are transmitted through all body tissues within an X-ray imaging region and are used to produce a black-and-white image that appears bright or dark according to the amount of X-ray photon attenuation that varies depending on the composition of tissue through which the X-rays pass. A medical X-ray imaging region includes skin, muscles, fat, bones, and various internal organs in a part of a body being imaged, such as the lungs, the abdominal organs, and the brain, and the organs have different X-ray transmittances. When an X-ray radiograph is captured, X-rays are emitted in one fixed direction and a brightness value shown in the X-ray radiograph is obtained as a single brightness value that is the sum of the effects of X-ray transmittances through various tissues positioned in a travel direction of X-ray photons. Thus, a brightness value of a specific tissue cannot be identified in an X-ray radiograph, and accordingly, an X-ray radiograph of the specific tissue cannot be obtained.
It is the technical problem underlying the invention to provide a method and an apparatus for reconstructing a medical image in an improved and novel manner.
The invention solves this problem by providing a method having the features of claim 1 and an apparatus having the features of claim 7.
This method and apparatus reconstruct a medical image of one or more tissues overlapping each other into a medical image of a specific issue by using an artificial intelligence (AI) model trained via deep learning, etc.
Advantageous embodiments of the invention are mentioned in the dependent claims, the wording of which is herewith incorporated into the description by reference.

One or more embodiments of the invention include a method and apparatus for reconstructing a medical image, such as an X-ray radiograph, showing various tissues overlapping one another into a medical image of a specific tissue or a medical image from which the specific tissue is removed.

Additional aspects will be set forth in part in the description which follows and, in part, will be apparent from the description, or may be learned by practice of the presented embodiments of the disclosure.

According to one or more embodiments of the invention, a method of reconstructing a medical image includes: generating at least one base image by reducing a dimensionality of a three-dimensional (3D) medical image; generating at least one segmented image by reducing a dimensionality of a 3D image of a region of a tissue segmented from the 3D medical image or a 3D image of a region excluding the tissue from the 3D medical image; and training, by using training data including the at least one base image and the at least one segmented image, an artificial intelligence (AI) model that separates at least one tissue from a medical image showing a plurality of tissues overlapping one another on the same plane.

According to one or more embodiments of the invention, an apparatus for reconstructing a medical image includes: a base image generator configured to generate at least one base image by reducing a dimensionality of a 3D medical image; a segmented image generator configured to generate at least one segmented image by reducing a dimensionality of a 3D image of a region of a tissue segmented from the 3D medical image or a 3D image of a region excluding the tissue from the 3D medical image; and a training unit configured to train, by using training data including the at least one base image and the at least one segmented image, an AI model that separates at least one tissue from a medical image showing a plurality of tissues overlapping one another on the same plane.

Advantageous embodiments of the invention are illustrated in the accompanying drawings. These and other advantageous embodiments of the invention are described in further detail in the following. In the drawings:
FIG. 1 illustrates an overall process of reconstructing a medical image, according to an embodiment;
FIG. 2 illustrates an example of a configuration of a medical image reconstruction apparatus according to an embodiment;
FIG. 3 illustrates a configuration of a training data generator of a medical image reconstruction apparatus, according to an embodiment;
FIG. 4 illustrates an example of a three-dimensional (3D) medical image according to an embodiment;
FIG. 5 illustrates an example of a method of reducing dimensionality of a medical image, according to an embodiment;
FIGS. 6A, 6B, and 7 respectively illustrate examples of application of an artificial intelligence (AI) model, according to an embodiment;
FIG. 8 illustrates an example of a medical image reconstructed using a trained AI model, according to an embodiment;
FIG. 9 is a flowchart of a method of reconstructing a medical image, according to an embodiment;
FIG. 10 is a flowchart of a method of reconstructing a two-dimensional (2D) medical image by using an AI model, according to an embodiment; and
FIG. 11 illustrates an example in which a medical image is reconstructed using an AI model, according to an embodiment.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Hereinafter, a method and apparatus for reconstructing a medical image according to embodiments will be described in detail with reference to the accompanying drawings.

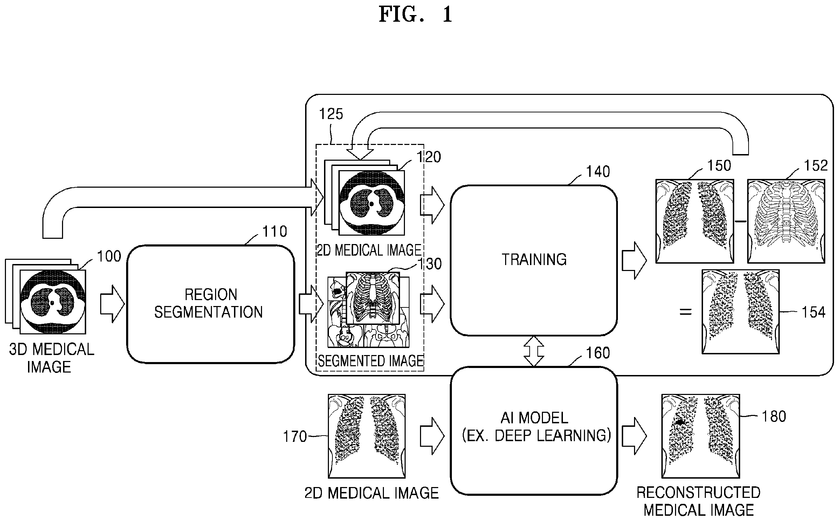

FIG. 1 illustrates an overall process of reconstructing a medical image, according to an embodiment.

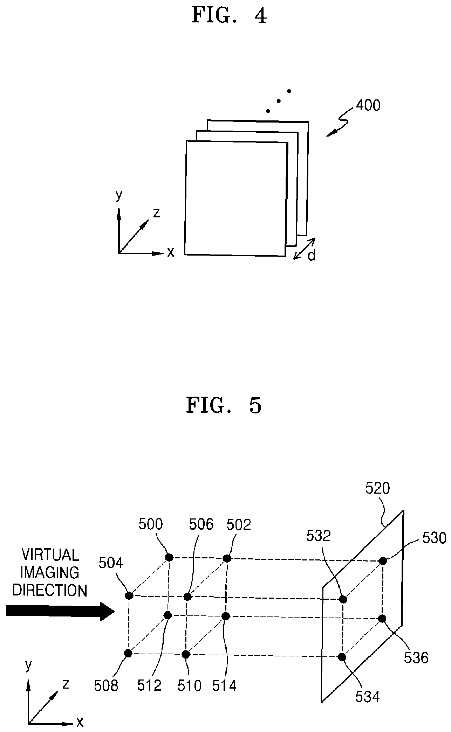

Referring to FIG. 1, training data 125 is used to train an artificial intelligence (AI) model 160 via deep learning, etc. and created by reducing the dimensionality of a 3D medical image 100. An example of a 3D medical image 100 is a computed tomography (CT) image (see FIG. 4) or a magnetic resonance imaging (MRI) image. The 3D medical image 100 according to the embodiment is not limited to a CT or MRI image, and includes any type of 3D image produced using various imaging modalities.

In the following embodiments including the present embodiment, a medical image generated by reducing the dimensionality of the 3D medical image 100 is referred to as a base image 120. Furthermore, a medical image generated by segmenting, from the 3D medical image 100, at least one tissue (e.g., soft tissues such as muscles, fat, the heart, blood vessels, etc., bones, various organs such as the lungs, the liver, etc., lesion tissue such as a tumor or a pulmonary nodule, etc.) (operation 110) and reducing the dimensionality of an image related to the segmented tissue is hereinafter referred to as a segmented image 130. The segmented image 130 may be an image obtained by reducing the dimensionality of a 3D image of a specific tissue (e.g., a 3D image including only a lung tissue) segmented from the 3D medical image 100, or an image obtained by reducing the dimensionality of a 3D image that is generated by removing a specific tissue (e.g., soft tissues such as muscles, fat, blood vessels, etc. or bone tissue) from the 3D medical image 100. An example of a method of reducing the dimensionality of a 3D image to a 2D image is shown in FIG. 5.

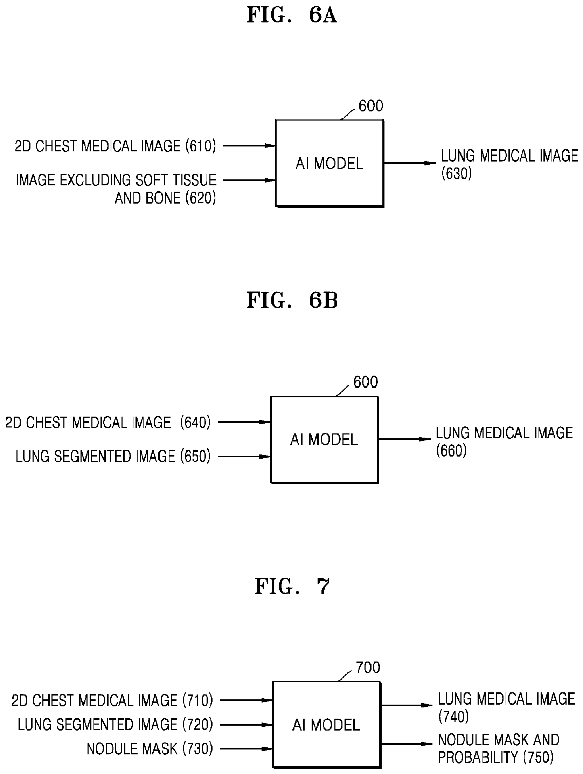

The base image 120 and the segmented image 130 are used for training 140 of the AI model 160. The AI model 160 may be implemented as various architectures such as convolution neural network (CNN), densely connected convolutional network (DenseNet), U-net, Goolenet, etc. For example, when the AI model 160 is implemented as a CNN, the AI model 160 may perform the training 140 for adjusting connection weights in an artificial neural network by using the training data 125. Examples of applying the AI model 160 according to an embodiment are shown in FIGS. 6A, 6B, and 7.

There are various techniques of the related art with respect to the AI model 160 itself. The embodiment does not relate to the AI model 160 itself but to a method and apparatus for training the AI model 160 implemented as various architectures of the related art to be used for reconstruction of a medical image according to the embodiment and a method and apparatus for separating a specific tissue from a medical image or for reconstructing a medical image related to the specific tissue by using the AI model trained according to an embodiment.

For example, a medical image reconstruction apparatus according to an embodiment may train the AI model 160 by using training data including the base image 120 and the segmented image 130 generated by reducing the dimensionality of the 3D medical image 100 to 2D, such that the AI model 160 may output a predicted segmented image 154 by removing specific tissue 152 from a base image 150. For example, the AI model 160 implemented as a CNN may perform a learning process for adjusting various parameters including connection weights in an artificial neural network by comparing the predicted segmented image 154 with the segmented image 130 in the training data 125.

As another example, by using the AI model 160 trained according to an embodiment, the medical image reconstruction apparatus may output a medical image 180 including only a specific tissue by removing one or more tissues from a 2D medical image 170, such as an X-ray radiograph, showing various tissues overlapping one another. For example, when the 2D medical image 170 is a chest X-ray radiograph, the medical image reconstruction apparatus may reconstruct the medical image 180 including only a lung tissue by removing soft tissues such as muscles, fat, the heart, blood vessels, etc., and bone tissue from the chest X-ray radiograph. As another example, the medical image reconstruction apparatus may reconstruct a medical image including only soft tissue or bone tissue from the chest X-ray radiograph. As another example, the medical image reconstruction apparatus may reconstruct the medical image 180 including only at least one specific tissue such as a lung tissue by extracting the at least one specific tissue from the chest X-ray radiograph instead of removing the same.

In this way, the medical image reconstruction may be performed by removing or extracting one or more tissues. However, for convenience of description, embodiments will be mainly described below with respect to a method of reconstructing a medical image by removing one or more tissues.

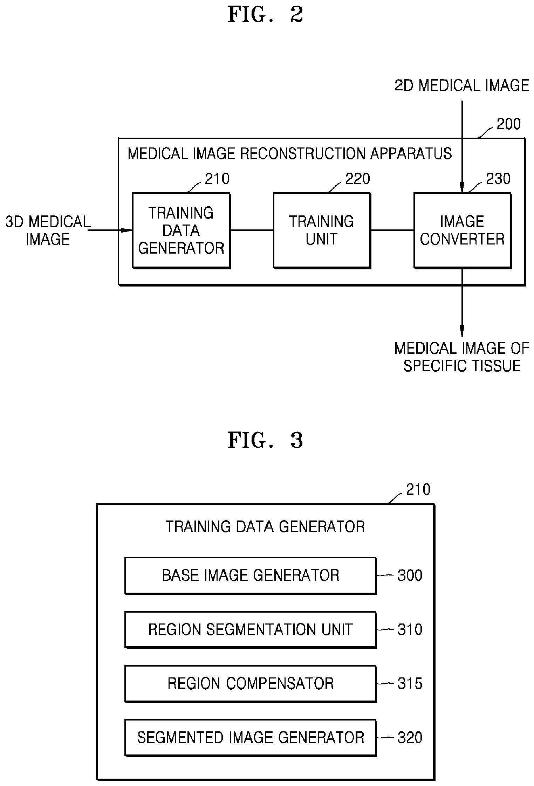

FIG. 2 illustrates an example of a configuration of a medical image reconstruction apparatus 200 according to an embodiment.

Referring to FIG. 2, the medical image reconstruction apparatus 200 includes a training data generator 210, a training unit 220, and an image converter 230. The medical image reconstruction apparatus 200 may be implemented as an apparatus including a memory, a processor, an input/output (I/O) device, etc., or as a software module loadable into a general computing device of the related art.

The training data generator 210 generates training data for an AI model. A 2D medical image such as an X-ray radiograph depicts various tissues overlapping one another. In other words, the brightness of a 2D medical image is affected by all tissues located along a path of X-ray transmission. For example, a chest X-ray radiograph shows a lung tissue, soft tissue of the chest, and bone tissue overlapping one another.

To train an AI model for separating a specific tissue from a 2D medical image, a 2D image obtained by separating the specific tissue from the 2D medical image is required for use as training data A user having expertise in anatomy may create training data by individually separating regions of specific tissues from a 2D medical image. However, because training of an AI model requires a large amount of training data, it is extremely difficult for the user to produce the training data one by one. Furthermore, when a region of each tissue is separated from a 2D medical image simply based on brightness, etc. the accuracy of tissue separation may be degraded.

Thus, in the embodiment, the training data generator 210 generates training data by using a 3D medical image containing more information than a 2D medical image. For example, the training data generator 210 may separate a region of specific tissue from a 3D medical image and generate training data by reducing the dimensionality of a 3D image of the separated region of specific tissue to 2D. For example., when the AI model is a model for reconstructing a lung X-ray radiograph from a chest X-ray radiograph and outputting the lung X-ray radiograph, the training data generator 210 may separate soft tissues and bone tissue from a 3D medical image of a chest and generate training data by reducing the dimensionality of a 3D image including only a lung tissue to 2D, the 3D image being obtained by removing the bone tissue and the soft tissues including muscles, fat, the heart, and blood vessels from the 3D medical image. Alternatively, the training data generator 210 may generate training data by reducing the dimensionality of a 3D image to 2D, the 3D image being extracted by selecting and segmenting only a lung tissue from the 3D medical image. An example of a detailed configuration of the training data generator 210 is shown in FIG. 3.

The training unit 220 trains the AI model by using training data generated by the training data generator 210.

The image converter 230 separates a specific tissue from a 2D medical image, such as an X-ray radiograph, showing various tissues overlapping one another, and reconstructs and outputs a medical image including the specific tissue or obtained by removing the specific tissue from the 2D medical image.

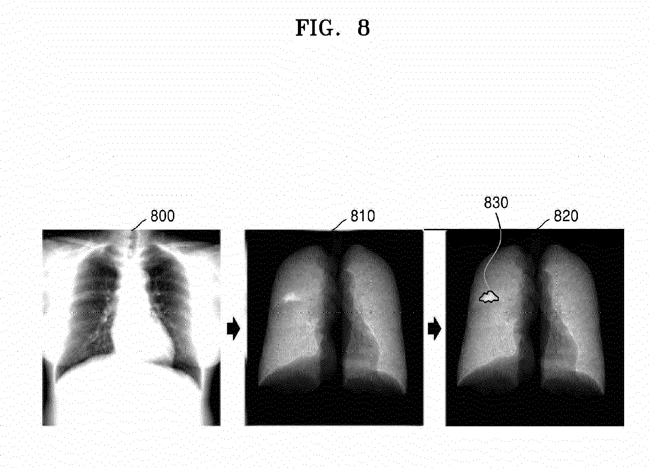

According to another embodiment, the medical image reconstruction apparatus 200 may train the AI model so as to identify lesion information in a medical image reconstructed to include specific tissue. This embodiment will be described in more detail below with respect to FIGS. 7 and 8.

FIG. 3 illustrates a configuration of an example of a training data generator 210 of a medical image reconstruction apparatus, according to an embodiment.

Referring to FIG. 3, the training data generator 210 includes a base image generator 300, a region segmentation unit 310, a region compensator 315, and a segmented image generator 320. The training data generator 210 may be implemented as a separate device. For example, the training data generator 210 may be implemented as an independent software module. In this case, a computing device including a memory, a processor, an I/O device, etc. may generate training data by loading and executing a software module implementing the training data generator 210. According to an embodiment, the training data generator 210 may not include the region compensator 315.

The base image generator 300 generates a base image by reducing a dimensionality of a 3D medical image. For example, the base image generator 300 may generate at least one 2D base image by projecting the 3D medical image in at least one direction. An example of a dimensionality reduction method is illustrated in FIG. 5.

The region segmentation unit 310 separates a region of specific tissue from the 3D medical image. The region segmentation unit 310 may separate a region of specific tissue from the 3D medical image by using various region segmentation algorithms of the related art. For example, the region segmentation unit 310 may segment a region of specific tissue from a 3D medical image by using segmentation methods described in Korean Patent Publication 10-1482247 titled "Method and Apparatus for Extracting Airways", Korean Patent Publication 10-1514003 titled "Method and Apparatus for Extracting Pulmonary Lobes", Korean Application Publication 10-2018-0098984 titled "Method and Apparatus for Segmenting Regions in Medical Image", etc. According to another embodiment, the region segmentation unit 310 may provide a user interface including various tools that a user uses to segment a region of specific tissue from the 3D medical image. According to another embodiment, when segmentation information regarding a tissue included in the 3D medical image is received from outside, the training data generator 210 may not include the region segmentation unit 310.

The region compensator 315 may fill the segmented region of specific tissue with a specific brightness value (e.g., a brightness value of pure water (20 Hounsfield units) or muscles, or a brightness value designated by the user).

The segmented image generator 320 may generate a segmented image by reducing the dimensionality of a 3D image of at least one tissue segmented from the 3D medical image. Alternatively, the segmented image generator 320 may generate a segmented image by reducing the dimensionality of a 3D medical image excluding at least one specific tissue from the 3D medical image. The segmented image generator 320 may generate at least one 2D segmented image by projecting a 3D image of the specific tissue or a 3D image from which the specific tissue is removed in at least one direction.

FIG. 4 illustrates an example of a 3D medical image 400 according to an embodiment.

Referring to FIG. 4, the 3D medical image 400 such as a CT image may include a plurality of x-y cross-sectional images taken at a specific interval d. The 3D medical image 400 may be composed of 3D voxels representing brightness and may be stored as a Digital Imaging and Communication in Medicine (DICOM) file.

FIG. 5 illustrates an example of a method of reducing dimensionality of a medical image according to an embodiment.

Referring to FIG. 5, a 3D medical image is composed of first through eighth voxels 500, 502, 504, 506, 508, 510, 512, and 514 respectively including their brightness values. In the embodiment, only the eight voxels, i.e., the first through eighth voxels 500, 502, 504, 506, 508, 510, 512, and 514 in the 3D medical image are shown in FIG. 5 for convenience of description.

A medical image reconstruction apparatus projects the 3D medical image onto a virtual plane 520 in a specific direction to reduce the dimensionality of a 3D medical image to 2D. An image that is a projection onto the virtual plane 520 is the 2D medical image. In this case, the medical image reconstruction apparatus obtains a brightness value of the 2D medical image by averaging brightness values of voxels overlapping each other in a projection direction. In detail, a brightness value of a 2D medical image such as an X-ray radiograph depends on one or more tissues located in an X-ray transmission direction. Thus, according to the embodiment, in order to generate, based on a 3D medical image, a 2D medical image, such as an X-ray radiograph, in which the effects on each tissue have been reflected, a brightness value of each pixel in the 2D medical image is obtained by averaging brightness values of voxels overlapping one another in a projection direction.

For example, when a virtual imaging direction (i.e., a projection direction) is parallel to an X-axis, the medical image reconstruction apparatus obtains a brightness value of a first pixel 530 in the 2D medical image that is a projection onto the virtual plane 520 by averaging brightness values of the first and second voxels 500 and 502 overlapping each other in the projection direction. Similarly, the medical image reconstruction apparatus obtains a brightness value of a second pixel 532 by averaging brightness values of the third and fourth voxels 504 and 506, a brightness value of a third pixel 534 by averaging brightness values of the fifth and sixth voxels 508 and 510, and a brightness value of a fourth pixel 536 by averaging brightness values of the seventh and eighth voxels 512 and 514. According to an embodiment, the medical image reconstruction apparatus may respectively generate 2D images with different projection directions based on a single 3D image.

FIGS. 6A, 6B, and 7 respectively illustrate examples of application of an AI model 600 according to an embodiment.

Referring to FIG. 6A, the AI model 600 is a model for outputting a lung medical image extracted from a 2D chest medical image. To achieve this, a medical image reconstruction apparatus generates a base image 610 by reducing the dimensionality of a 3D medical image of the chest to 2D, generates a segmented image 620 by removing soft tissues including muscles, fat, the heart, and blood vessels and bone tissue from the 3D medical image and then reducing the dimensionality of a medical image including a lung tissue, and inputs the base image 610 and the segmented image 620 as training data for the AI model 600. The AI model 600 generates a predicted segmented image 630 by removing soft tissue and bone tissue from the base image 610 and performs a learning process for adjusting various parameters by comparing the received segmented image 620 with the predicted segmented image 630.

As another example, referring to FIG. 6B, the medical image reconstruction apparatus may generate a base image 640 by reducing the dimensionality of a 3D medical image of the chest to 2D, generates a segmented image 650 by segmenting and extracting only a lung tissue from the 3D medical image and reducing the dimensionality of a medical image including the lung tissue, and uses the base image 640 and the segmented image 650 as training data. In this case, an AI model 600 may generate a predicted segmented image 660 by extracting only the lung tissue from the base image 640.

When a chest X-ray radiograph is received, the AI model 600 trained in this way reconstructs a medical image including only the lung tissue by removing soft tissues including muscles, fat, the heart, and blood vessels and bone tissue from the chest X-ray radiograph and outputs the reconstructed medical image. Because an X-ray radiograph is composed of 2D pixels, when pixels in soft tissues and bone tissue are simply removed, information about a lung tissue in a region where the soft tissues and the bone tissue are positioned is also removed. However, according to the embodiment, the AI model 600 is trained using a segmented image generated by removing soft tissues including muscles, fat, the heart, and blood vessels and bone tissue from a 3D medical image and then reducing the dimensionality of a 3D image including only a lung tissue. Thus, the AI model 600 may identify regions of soft tissues including muscles, fat, the heart, and blood vessels and bone tissue in an X-ray radiograph and output a medical image including only a lung tissue but excluding the soft tissues and bone tissue by removing only the effects of brightness values due to the soft tissues and the bone tissue from brightness values of identified regions.

Referring to FIG. 7, an AI model 700 is a model for not only outputting a medical image by removing soft tissues and bone tissue from a 2D chest X-ray radiograph but also detecting a suspicious pulmonary nodule region located within a lung in a lung radiograph excluding the soft tissues and bone tissue and outputting a probability of a suspicious pulmonary nodule.

For training of the AI model 700, the medical image reconstruction apparatus generates a base image 710 by reducing the dimensionality of a 3D medical image of the lung to 2D as described with reference to FIG. 6A or 6B and generates a segmented image 720 by reducing the dimensionality of a medical image including only a lung tissue after removing soft tissues including muscles, fat, the heart, and blood vessels and bone tissue from the 3D medical image. Furthermore, the medical image reconstruction apparatus generates mask information 730 indicating a nodule region of the lung in the segmented image 720, which is identified based on the 3D medical image. The mask information 730 indicating the nodule region may include various pieces of feature information such as a shape, a size, histogram, and texture of the nodule region represented in the segmented image 720. A lesion region such as the nodule region may be designated by a user in the 3D medical image or be automatically detected using various detection algorithms of the related art, and then various pieces of feature information about the lesion region may be extracted.

The medical image reconstruction apparatus inputs the base image 710, the segmented image 720, and the mask information 730 as training data for the AI model 700. The AI model 700 generates a predicted segmented image 740, including only a lung tissue by removing soft tissues including muscles, fat, the heart, and blood vessels and bone tissue from the base image 710, and a mask for a suspicious nodule in the lung and a probability of the suspicious nodule (briefly, referred to as a 'nodule mask and probability 750'), and performs a learning process for adjusting parameters by respectively comparing the predicted segmented image 740 and the nodule mask and probability 750 with the segmented image 720 and the mask information 730 in the training data.

When a chest X-ray radiograph is received, the trained AI model 700 may reconstruct a medical image including only a lung tissue by removing various soft tissues and bone tissue from the chest X-ray radiograph and output the reconstructed medical image. Furthermore, the AI model 700 may extract a suspicious pulmonary nodule region from the reconstructed medical image and output a probability of pulmonary nodule.

FIG. 8 illustrates an example of a medical image reconstructed using a trained AI model, according to an embodiment.

Referring to FIG. 8, when a chest X-ray radiograph 800 is received, an AI model generates a medical image 810 by removing various soft tissues and bone tissue from the chest X-ray radiograph 800. The AI model detects a suspicious pulmonary nodule region 830 in the radiograph 810 including only a lung tissue but excluding the various soft tissues and bone tissue and outputs a probability of suspicious pulmonary nodule.

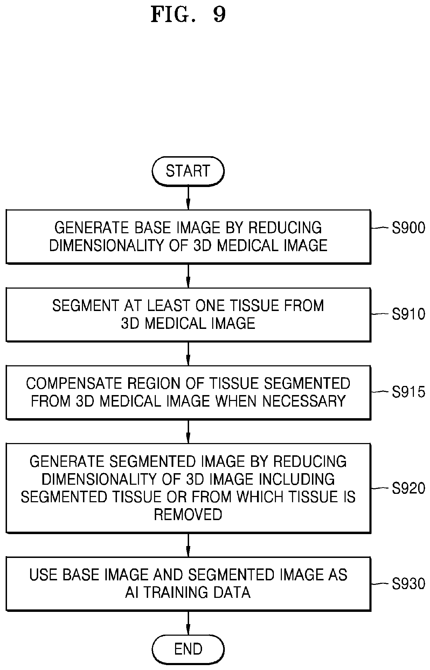

FIG. 9 is a flowchart of a method of reconstructing a medical image, according to an embodiment.

Referring to FIG. 9, a medical image reconstruction apparatus (hereinafter, referred to as an 'apparatus') generates a base image by reducing the dimensionality of a 3D medical image (S900). The apparatus segments one or more tissues from the 3D medical image (S910). Segmentation of a specific tissue from the 3D medical image (S910) may be performed via an automated algorithm or a user's manual operation. Furthermore, when necessary, the apparatus may regularly fill a region of the segmented tissue with a specific brightness value (S915). Then, the apparatus generates a segmented image by reducing the dimensionality of a 3D image of the segmented tissue or a 3D image obtained by removing the specific tissue from the 3D medical image (S920). The apparatus trains an AI model that separates at least one tissue from a medical image showing a plurality of tissues overlapping one another on the same plane by using the base image and the segmented image as training data (S930).

According to another embodiment, the apparatus may train the AI model by using, as training data, values of analysis including a histogram or texture for a lesion tissue together with the base image and the segmented image. For example, the AI model as described with reference to FIG. 7 may be trained to automatically detect a pulmonary nodule in an X-ray radiograph.

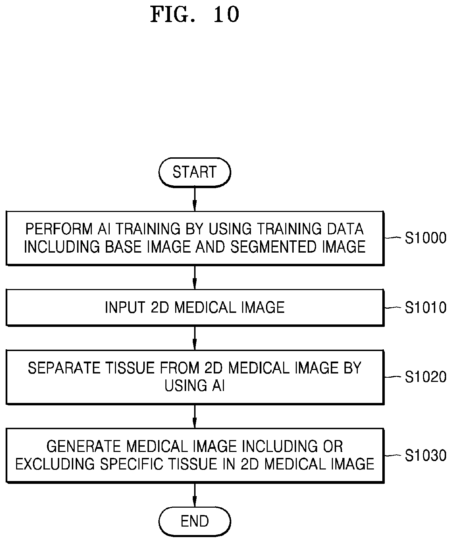

FIG. 10 is a flowchart of a method of reconstructing a 2D medical image by using an AI model, according to an embodiment.

Referring to FIG. 10, a medical image reconstruction apparatus trains an AI model by using the method described with reference to FIG. 9 (S1000). When a 2D medical image such as an X-ray radiograph is input (S1010), the medical image reconstruction apparatus separates a specific tissue from the 2D medical image by using the trained AI model (S1020) and reconstructs and outputs a 2D radiograph including only the separated specific tissue or from which the separated specific tissue is removed (S1030).

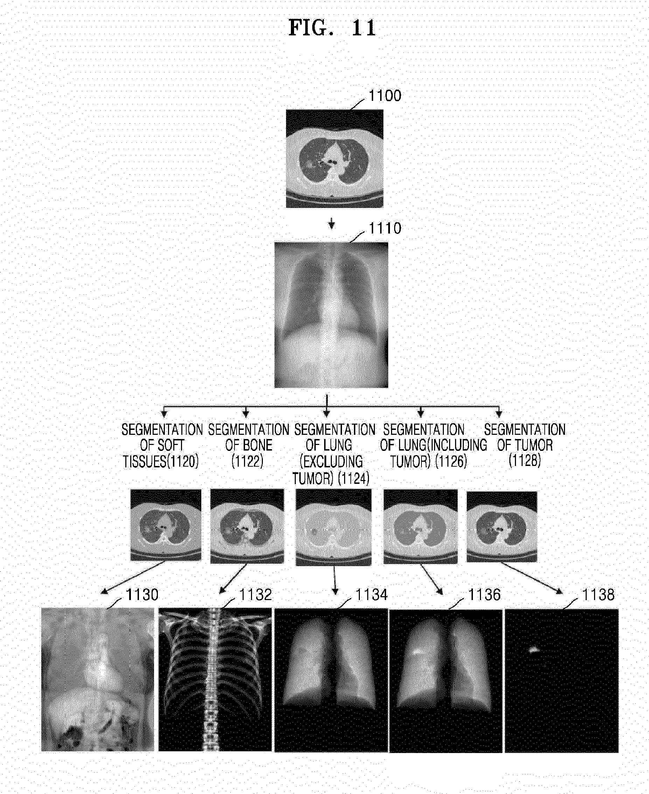

FIG. 11 illustrates an example in which a medical image is reconstructed using an AI model, according to an embodiment.

Referring to FIG. 11, a medical image reconstruction apparatus generates a base image 1110 that is a dimensionally reduced version of a 3D chest CT image 1100 as training data for an AI model. Furthermore, the medical image reconstruction apparatus segments specific tissues from the 3D chest CT image 1100 (1120, 1122, 1124, 1126, and 1128) according to the type of tissue of which a medical image is to be reconstructed using the AI model and generates a segmented image as training data via dimensionality reduction.

For example, when the AI model is a model for reconstructing a medical image 1130 including only soft tissues such as muscles, fat, the heart, blood vessels, etc. from the 3D chest CT image 1100, a segmented image in the training data is obtained by segmenting the soft tissues from the 3D chest CT image 1100 and reducing the dimensionality of an image related to the segmented soft tissues. As another example, when the AI model is a model for reconstructing a medical image 1132 including only a bone tissue from the 3D chest CT image 1100, a segmented image is obtained by segmenting the bone tissue (1122) and reducing the dimensionality of a 3D medical image related to the segmented bone tissue. As another example, the AI model may be a model for reconstructing a medical image 1134 including only a lung tissue, a medical image 1136 including the lung tissue and a tumor, or a medical image 1138 including only a pulmonary nodule. In each case, a segmented image may be obtained by segmenting the lung tissue (1124), the lung tissue and tumor (1126), or only the tumor (1128), and reducing the dimensionality of a 3D image related to the segmented lung tissue, lung tissue and tumor, or tumor.

As described above, the medical image reconstruction apparatus according to the embodiment may reconstruct a medical image including only at least one tissue such as soft tissue, bone tissue, lung tissue, etc. from an X-ray radiograph.

Medical image reconstruction methods according to embodiment may be embodied as a computer-readable code on a computer-readable recording medium. The computer-readable recording medium is any data storage device for storing data which can be thereafter read by a computer system. Examples of computer-readable recording media include read-only memory (ROM), random-access memory (RAM), compact disc (CD)-ROMs, magnetic tapes, floppy disks, optical data storage devices, etc. The computer-readable recording media may also be distributed over network-coupled computer systems so that computer-readable codes are stored and executed in a distributed fashion.

According to embodiments of the disclosure, it is possible to reconstruct a medical image, such as an X-ray radiograph, showing various tissues overlapping one another into a medical image including a specific tissue or from which the specific tissue is removed. Furthermore, it is possible to automate reconstruction of a medical image by using an AI model trained via deep learning, etc. and improve the result of training of the AI model by specifying training data for the AI model in detail via dimensionality reduction of a 3D medical image. According to another embodiment, when an AI model is trained with respect to information about a lesion, the lesion may be detected in a medical image including limited information, such as an X-ray radiograph.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope of the disclosure as defined by the following claims.

## Claims

1. A method of reconstructing a medical image, the method comprising:
generating at least one base image by reducing a dimensionality of a three-dimensional medical image;
generating at least one segmented image by reducing a dimensionality of a three-dimensional image of a region of a tissue segmented from the three-dimensional medical image or a three-dimensional image of a region excluding the tissue from the three-dimensional medical image; and
training, by using training data including the at least one base image and the at least one segmented image, an artificial intelligence (AI) model that separates at least one tissue from a medical image showing a plurality of tissues overlapping one another on the same plane.

2. The method of claim 1, further comprising:
receiving a two-dimensional medical image; and
separating a specific tissue from the two-dimensional medical image via the AI model and generating a medical image including the specific tissue or a medical image from which the specific tissue is removed.

3. The method of claim 2, wherein
the three-dimensional medical image includes a computed tomography (CT) image or a magnetic resonance imaging (MRI) image, and
the two-dimensional medical image includes an X-ray radiograph.

4. The method of any one of claims 1 to 3, wherein
the generating of the at least one base image comprises generating at least one two-dimensional base image by projecting the three-dimensional medical image in at least one direction, and
the generating of the at least one segmented image comprises generating at least one two-dimensional segmented image by projecting the three-dimensional image of the region of the tissue or the three-dimensional image of the region excluding the region of the tissue in at least one direction.

5. The method of any one of claims 1 to 4, wherein the training of the AI model comprises training the AI model by using training data further including a value of analysis including a histogram or texture for a lesion tissue.

6. The method of any one of claims 1 to 5, further comprising filling the region of the tissue segmented from the three-dimensional medical image with a specific brightness value.

7. An apparatus for reconstructing a medical image, the apparatus comprising:
a base image generator (300) configured to generate at least one base image by reducing a dimensionality of a three-dimensional medical image;
a segmented image generator (320) configured to generate at least one segmented image by reducing a dimensionality of a three-dimensional image of a region of a tissue segmented from the three-dimensional medical image or a three-dimensional image of a region excluding the tissue from the three-dimensional medical image; and
a training unit (200) configured to train, by using training data including the at least one base image and the at least one segmented image, an artificial intelligence (AI) model that separates at least one tissue from a medical image showing a plurality of tissues overlapping one another on the same plane.

8. The apparatus of claim 7, further comprising a region segmentation unit (310) configured to segment at least one tissue from the three-dimensional medical image.

9. The apparatus of claim 7 or 8, further comprising an image converter (230) configured to separate a specific tissue from a two-dimensional medical image via the AI model and generate a medical image including the specific tissue or a medical image from which the specific tissue is removed.

10. The apparatus of any one of claims 7 to 9, wherein
the base image generator (300) is further configured to generate at least one two-dimensional base image by projecting the three-dimensional medical image in at least one direction, and
the segmented image generator (300) is further configured to generate at least one two-dimensional segmented image by projecting the three-dimensional image of the region of the tissue or the three-dimensional image of the region excluding the region of the tissue in at least one direction.

11. The apparatus of any one of claims 7 to 10, wherein the training unit (220) is further configured to train the AI model by using training data further including a value of analysis including a histogram or texture for a lesion tissue.

12. The apparatus of any one of claims 7 to 11, further comprising a region compensator (315) configured to fill the region of the tissue segmented from the three-dimensional medical image with a specific brightness value.

13. A computer-readable recording medium having recorded thereon a program code for performing the method of any of claims 1 to 6.
